(19) Europäisches Patentamt
European Patent Office
Office européen des brevets

(11) Veröffentlichungsnummer: **0 514 692 A2**

(12) # EUROPÄISCHE PATENTANMELDUNG

(21) Anmeldenummer: **92107265.8**

(51) Int. Cl.5: **C07C 209/14**, C07C 209/26

(22) Anmeldetag: **29.04.92**

(30) Priorität: **18.05.91 DE 4116367**

(43) Veröffentlichungstag der Anmeldung:
**25.11.92 Patentblatt 92/48**

(84) Benannte Vertragsstaaten:
**BE DE FR GB NL**

(71) Anmelder: **BASF Aktiengesellschaft**
**Carl-Bosch-Strasse 38**
**W-6700 Ludwigshafen(DE)**

(72) Erfinder: **Hartig, Juergen, Dr.**
**In der Schleit 9**
**W-6718 Gruenstadt(DE)**
Erfinder: **Schnur, Rudolf**
**Worthington Ave. 11161**
**Baton Rouge, LA 70815(US)**
Erfinder: **Schroeder, Wolfgang, Dr.**
**Seebacher Strasse 51**
**W-6702 Bad Duerkheim(DE)**

(54) **Verfahren zur Herstellung von Aminen.**

(57) Verfahren zur Herstellung von Aminen der allgemeinen Formel I

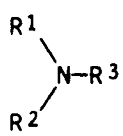

(I),

in der

- $R^1$ Wasserstoff oder $R^2$,
- $R^2$ $C_1$- bis $C_{12}$-Alkyl, $C_3$- bis $C_{12}$-Cycloalkyl, $C_2$- bis $C_{12}$-Alkoxyalkyl, gegebenenfalls durch $C_1$- bis $C_8$-Alkyl und/oder $C_1$- bis $C_8$-Alkoxy substituiertes Aryl oder $C_7$- bis $C_{12}$-Aralkyl,
- $R^3$ Wasserstoff oder $C_1$- bis $C_{12}$-Alkyl

bedeutet, durch Umsetzung von Alkanolen der allgemeinen Formel II

$R^2$-OH (II),

in der $R^2$ die oben genannten Bedeutungen hat, mit Ammoniak oder einem primären Amin der allgemeinen Formel III

$R^3$-$NH_2$ (III),

in der $R^3$ die oben genannten Bedeutungen hat, und einem Katalysator bei Temperaturen von 100 bis 250 °C und Drücken von 1 bis 100 bar, indem man in der Gasphase arbeitet und Katalysatoren einsetzt, deren katalytisch aktive Masse 5 bis 100 Gew.-% eines Oxides von Kupfer und Nickel im Atomverhältnis von 1:1 bis 10:1 und Zirkon- und/oder Aluminiumoxid enthält.

EP 0 514 692 A2

Die vorliegende Erfindung betrifft ein neues und verbessertes Verfahren zur Herstellung von Aminen durch Umsetzung von Alkanolen und Ammoniak oder primären Aminen bei erhöhten Temperaturen und erhöhten Drücken in der Gasphase in Gegenwart eines Katalysators, dessen katalytisch aktive Masse 5 bis 100 Gew.-% eines Oxides von Kupfer und Nickel im Atomverhältnis von 1:1 bis 10:1 und Zirkon- und/oder Aluminiumoxid enthält.

Aus der EP-A-382 049 ist ein Verfahren zur Herstellung von Aminen aus Alkoholen und Ammoniak bekannt, das in der Flüssigphase an Katalysatoren die $ZrO_2$, CuO und CoO bzw. NiO enthalten, in der Sumpf- oder Rieselfahrweise durchgeführt wird. Hierbei waren die Raum-/Zeit-Ausbeuten bzw. die Selektivität verbesserungsbedürftig.

Ferner ist aus der EP-A-167 872 ein Verfahren zur Herstellung von tert.-Aminen bekannt, in denen Dimethylamin mit Alkoholen in der Gasphase an kupferhaltigen Katalysatoren umgesetzt wurde.

Aus der EP-A-70 397 ist ein Gasphasenverfahren zur Herstellung cyclischer Imine an Katalysatoren, die Kupfer und eine geringe Menge Nickel und Aluminiumoxid enthalten, bekannt.

Der vorliegenden Erfindung lag die Aufgabe zugrunde, den Nachteilen des Standes der Technik abzuhelfen.

Demgemäß wurde ein neues und verbessertes Verfahren zur Herstellung von Aminen der allgemeinen Formel I

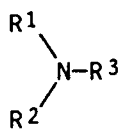

(I),

in der

$R^1$ - Wasserstoff oder $R^2$,

$R^2$ - $C_1$- bis $C_{12}$-Alkyl, $C_3$- bis $C_{12}$-Cycloalkyl, $C_2$- bis $C_{12}$-Alkoxyalkyl, gegebenenfalls durch $C_1$- bis $C_8$-Alkyl und/oder $C_1$- bis $C_8$-Alkoxy substituiertes Aryl oder $C_7$- bis $C_{12}$-Aralkyl,

$R^3$ Wasserstoff oder $C_1$- bis $C_{12}$-Alkyl

bedeutet, durch Umsetzung von Alkanolen der allgemeinen Formel II

$R^2$-OH (II),

in der $R^2$ die oben genannten Bedeutungen hat, mit Ammoniak oder einem primären Amin der allgemeinen Formel III

$R^3$-$NH_2$ (III),

in der $R^3$ die oben genannten Bedeutungen hat, und einem Katalysator bei Temperaturen von 100 bis 250 °C und Drücken von 1 bis 100 bar, gefunden, welches dadurch gekennzeichnet ist, daß man in der Gasphase arbeitet und Katalysatoren einsetzt, deren katalytisch aktive Masse 5 bis 100 Gew.-% eines Oxides von Kupfer und Nickel im Atomverhältnis von 1:1 bis 10:1 und Zirkon- und/oder Aluminiumoxid enthält.

Das erfindungsgemäße Verfahren läßt sich wie folgt durchführen:

Das Alkanol II und Ammoniak bzw. primäre Amine III werden gasförmig bei Drücken von 1 bis 100 bar, bevorzugt 1,5 bis 50 bar und bei Temperaturen zwischen 100 °C und 250 °C, besonders zwischen 130 °C und 220 °C über einen Katalysator geleitet, dessen aktive Masse Kupfer und Nickel im Atomverhältnis von 1:1 bis 10:1, bevorzugt von 2:1 bis 5:1, sowie Zirkon- und/oder Aluminiumoxid enthält.

Zur Erhaltung der Gasphase, d.h. zur Verhinderung von partieller Kondensation, werden die Reaktionsteilnehmer in einem Wasserstoff- bzw. Stickstoffstrom verdampft. Nach dem Kondensieren der Reaktionsprodukte können Teile der Edukte in den Verdampferteil zurückgeführt werden.

Das Reaktorsystem zur Durchführung der Synthese besteht im allgemeinen aus den Vorratsbehältern für die Reaktionsteilnehmer, aus denen diese in einen Vorheizer gepumpt werden, die dann gemeinsam mit dem Wasserstoff und/oder Stickstoff in den Verdampfer gelangen. Hier werden sie auf eine Temperatur gebracht, die bevorzugt etwas oberhalb der Synthesetemperatur liegt, z.B. 10 °C höher. Die Reaktion wird vorteilhafterweise isotherm geführt.

Als Reaktortyp eignen sich alle für solche Reaktionen übliche Reaktoren, besonders eine Wirbelschicht mit Einbauten zur Wärmeabfuhr oder Röhrenreaktoren.

Diese können beispielsweise durch eine Siedekühlung weitgehend isotherm gehalten werden, wobei die Temperatur des Kühlmediums durch die entsprechende Regulierung seines Dampfdruckes eingestellt werden kann.

Bei Verwendung einer Wirbelschicht wird der Katalysator bevorzugt in feinteiliger Form mit Teilchendurchmesser im Bereich um 0,1 mm eingesetzt. Bei Verwendung eines Röhrenreaktors sind Partikeln mit Durchmessern von 2 bis 6 mm zweckmäßig. Alle gebräuchlichen geometrischen Formen (Tabletten, Stränge, Kugeln, Ringe usw.) können benutzt werden.

Die gasförmig den Reaktor verlassenden Reaktionsprodukte werden einstufig, bevorzugt zweistufig kondensiert, wobei in der ersten Stufe die Wärme auf das zum Reaktor strömende Gemisch der Reaktionsteilnehmer übertragen wird. In der zweiten Stufe wird in üblicher Weise die Kondensation vervollständigt. Das Kondensat wird aus dem Reaktorsystem abgezogen und aufgearbeitet. In vielen Fällen wird nur eines der Amine benötigt, beispielsweise das primäre Amin. Im Allgemeinen entsteht auch das sekundäre, gegebenenfalls auch das tertiäre Amin. Außerdem enthält das Kondensat nicht umgesetzten Alkohol. Soweit diese Stoffe verdampflich sind, werden sie zusammen mit dem frischen Zulauf (Alkohol) wieder dem Reaktor zugeführt. Weil die Reaktion im allgemeinen bis zum Gleichgewicht verläuft, erhält man auf diesem Weg vorzugsweise das gewünschte Amin.

Als Katalysatoren für das erfindungsgemäße Verfahren eignen sich solche Katalysatoren, deren aktive Masse Kupfer, Nickel und/oder Kobalt, sowie Zirkon und/oder Aluminium in Form von Oxiden enthalten. Am besten geeignet sind Katalysatoren, wie sie z.B. in EP-A-70 379 und der EP-A-167 872 beschrieben wurden. Bevorzugt werden Katalysatoren, deren katalytisch aktive Masse 20 bis 80 Gew.-%` besonders 40 bis 70 Gew.-% $Al_2O_3$ und/oder $ZrO_2$, 1 bis 30 Gew.-% Kupferoxid als Kupfer-II-oxid, 1 bis 30 Gew.-% Nickeloxid und gegebenenfalls 1 bis 30 Gew.-% Kobaltoxid enthalten.

Die Brutto-Zusammensetzung liegt also in den Bereichen: 40 bis 80 % $Al_2O_3$ oder $ZrO_2$; 54 bis 10 % Cu; 24 bis 2 % Nickel. Ein Teil des Nickels kann durch Kobalt ersetzt werden. Die Herstellung erfolgt durch gemeinsame Fällung aus einer Mischlösung der Nitrate mittels Sodalösung oder einer Mischlösung von Soda und Natriumhydroxid. Die Fällung wird von der Mutterlauge getrennt, gewaschen, zu Partikeln verformt und bei einer zwischen 300 und 650° liegenden Temperatur getempert. Vor der Durchführung der Reaktion wird der Katalysator mit Wasserstoff, gegebenenfalls in einer Mischung mit Stickstoff, durch Reduzieren des Kupfers, Nickels und gegebenenfalls Kobalts bis zum Metall aktiviert. Die dafür anzuwendenden Temperaturen reichen von 150°C bis 300°C.

Die durch dieses Verfahrensprinzip und den dabei eingesetzten Katalysator erreichten Vorteile sind z.B.

- Aminierungsreaktion bis zum Gleichgewicht
- im allgemeinen lineare Beziehungen zwischen der Ammoniakkonzentration und der Konzentration der Reaktionsprodukte.

Hiermit wird die Einstellung vorgegebener Produktverhältnisse erleichtert. Desgleichen wird die Rückführung und Umsetzung unerwünschter Produkte zum jeweiligen Zielprodukt vereinfacht.

Die Substituenten $R^1$, $R^2$ und $R^3$ in den Verbindungen I, II und III haben folgende Bedeutungen:

$R^1$

- Wasserstoff,
- Bedeutungen des Restes $R^2$,

$R^2$

- $C_1$- bis $C_{12}$-Alkyl, bevorzugt $C_1$- bis $C_{12}$-Alkyl wie Methyl, Ethyl, n-Propyl, iso-Propyl, n-Butyl, iso-Butyl, sec.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt $C_2$- bis $C_8$-Alkyl wie Methyl, Ethyl, n-Propyl, iso-Propyl, n-Butyl, iso-Butyl, sec.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl und n-Octyl,
- $C_1$- bis $C_{12}$-Cycloalkyl, bevorzugt $C_3$- bis $C_8$-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- $C_2$- bis $C_{12}$-Alkoxyalkyl, bevorzugt $C_2$- bis $C_8$-Alkoxyalkyl wie Methoxymethyl, Ethoxymethyl, n-Propoxymethyl, iso-Propoxy-methyl, n-Butoxymethyl, iso-Butoxymethyl, sec.-Butoxymethyl, tert.-Butoxymethyl, 1-Methoxy-ethyl und 2-Methoxy-ethyl, besonders bevorzugt $C_1$-bis $C_4$-Alkoxyalkyl wie Methoxiethyl, Methoxipropyl und Methoxiisopropyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- durch $C_1$- bis $C_4$-Alkyl und/oder $C_1$- bis $C_4$-Alkoxy substituiertes Aryl,

- $C_7$- bis $C_{12}$-Aralkyl, bevorzugt $C_7$- bis $C_{12}$-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- durch $C_1$- bis $C_4$-Alkyl und/oder $C_1$- bis $C_4$-Alkoxy und/oder Halogen ein- bis dreifach substituiertes $C_7$- bis $C_{12}$-Aralkyl.

$R^3$

- Wasserstoff,
- $C_1$- bis $C_{12}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl.

Geeignete Amine der allgemeinen Formel I und Alkanole der allgemeinen Formel II sind z.B.:

| Amine I | Alkanole II |
|---|---|
| n-Propylamin | n-Propanol |
| iso-Propylamin | iso-Propanol |
| n-Butylamin | n-Butanol |
| iso-Butylamin | iso-Butanol |
| sec.-Butylamin | sec.-Butanol |
| Pentylamine | Pentanole |
| Hexylamine | Hexanole |
| Heptylamine | Heptanole |
| Octylamine | Octanole |

Die nach dem erfindungsgemäßen Verfahren herstellbaren Amine der allgemeinen Formel I sind Zwischenprodukte für die Herstellung von Tensiden, Arzneimitteln und Wirkstoffen für den Pflanzenschutz sowie für die Herstellung von Vulkanisierungsbeschleunigern.

## Beispiele

### Beispiel 1

Herstellung von Mono-sekundär-Butylamin

Es wurde eine Apparatur verwendet, die aus folgenden Teilen bestand: Vorratsgefäß für sekundär-Butanol, Dosierpumpe, Vorheizer; Vorratsgefäß für Ammoniak, Dosierpumpe für Ammoniak, Verdampfer für sekundär-Butanol, Röhrenreaktor, Kühler, Kondensatabscheider, Kreisgaskompressor. Zwischen dem Kondensatabscheider und der Saugseite des Kompressors befindet sich eine Vorrichtung zum Ableiten von Abgas und zu seiner mengenmäßigen und analytischen Kontrolle. Die Zuleitung von frischem Wasserstoff zur Ergänzung der Verluste (Abgas und Lösung im Kondensat) befindet sich unmittelbar vor dem Verdampfer für sekundär-Butanol. Der Reaktor besteht aus einem zylindrischen Rohr mit 45 mm Innendurchmesser, das von einem mit Wärmeträgeröl durchflossenen Mantel umgeben ist. Im Reaktionsrohr befindet sich auf einer Lage von Raschigringen aus Edelstahl die Katalysatorfüllung von 1 Liter. Sie besteht (nach der Aktivierung) aus 55 Gew.-% $Al_2O_3$, 36 Gew.-% Kupfer und 7 Gew.-% Nickel. Oberhalb der Katalysatorfüllung befindet sich eine weitere Lage von Raschigringen. Die Aktivierung wird begonnen bei 170 °C mit einem Gasgemisch aus 90 Vol.-% Stickstoff und 10 Vol.-% Wasserstoff. Dabei wird der Kupferanteil weitgehend vom ursprünglich oxidischen Zustand zum Metall reduziert.

Diese Aktivierung wird bei atmosphärischem Druck ausgeführt. Nach ihrer Beendigung wird die Temperatur auf 280° erhöht, um auch den Nickelanteil zum Metall zu reduzieren. Danach wird die Temperatur des umlaufenden Wärmeträgermediums auf 200° gesenkt.

Nunmehr wird der Druck im Reaktorsystem auf 16 bar (absolut) erhöht, der Kreisgaskompressor in Betrieb genommen, und die Kreisgasmenge von 440 Liter Gas pro Liter Katalysator und pro Stunde

eingestellt (gemessen bei 20 ° C und 16 bar).

Bei Konstanthaltung der Kreisgasmenge wird dem Wasserstoff Ammoniak beigemischt, bis seine Konzentration 45 Vol.-% beträgt. Dann beginnt die Zufuhr von sekundärem Butanol, das einen Wertstoffgehalt von 99,6 % hat. Die Zuflußmenge wird auf 1 Liter pro Stunde eingestellt. Durch die Reaktionswärme erhöht sich im Reaktor die Temperatur um 14 ° über diejenige der Thermostatierflüssigkeit im Reaktormantel.

Die gasförmigen Reaktionsprodukte werden in einem Kühler kondensiert, in einem Druckabschneider vom Kreisgas getrennt, und nach Entspannen auf Atmosphärendruck gesammelt. Das Kreisgas wird mit Hilfe des Kreisgaskompressors zum S-Butanolverdampfer zurückgefördert.

Die Untersuchung des Kondensats erfolgt sowohl durch fraktionierende Destillation als auch durch gaschromatographische Analyse. Während eines mehrwöchigen kontinuierlichen Versuches hat das Kondensat die folgende mittlere Zusammensetzung:

| | |
|---|---|
| Monosekundärbutylamin | 89,4 % |
| Di-sekundärbutylamin | 8,1 % |
| Sekundärbutanol | 2,0 % |
| Nebenprodukte | 0,5 % |

Beispiele 2 bis 4

Es wurde vorgegangen wie im Beispiel 1, jedoch mit den Unterschieden, daß die Ammoniakkonzentration im Kreisgas schrittweise vermindert wurde. Nach jeweils mehrwöchigen kontinuierlichen Versuchen wurden die folgenden Kondensatzusammensetzungen gefunden:

| Beispiel Nr. | Ammoniak im Kreisgas (Vol.-%) | Sekundär-Butylamin | | sek.-Butanol | Nebenprodukte |
|---|---|---|---|---|---|
| | | Mono- | Di- | | |
| 2 | 40 | 87,7 | 9,1 | 2,6 | 0,6 |
| 3 | 30 | 83,9 | 12,1 | 3,5 | 0,5 |
| 4 | 20 | 79,8 | 15,0 | 4,7 | 0,5 |

Die Beispiele 1 bis 4 zeigen die linearen Abhängikeiten der Stoffkonzentrationen von der Ammoniakkonzentration.

Beispiel 5

Es wurde vorgegangen wie in Beispiel 1, jedoch mit dem Unterschied, daß das sekundär-Butanol 10 Gew.-% Di-Sekundärbutylamin enthielt.

Das Kondensat hatte nach einwöchigem kontinuierlichem Betrieb folgende Zusammensetzung:

| | |
|---|---|
| Monosekundärbutylamin | 89,2 % |
| Di-sekundärbutylamin | 8,6 % |
| Sekundärbutanol | 1,8 % |
| Nebenprodukte | 0,4 % |

Aus den Ergebnissen der Beispiele 1 und 5 wird der Schluß auf Gleichgewichtseinstellung gezogen.

Beispiel 6 (Vergleichsbeispiel)

Es wird vorgegangen wie in Beispiel 4, jedoch mit dem Unterschied, a) daß ein Katalysator mit 65 Gew.-% Nickel verwendet wurde und b) die Temperatur des Wärmeträgermediums 190 ° C betrug. Das Kondensat hatte nach mehrwöchigem kontinuierlichem Betrieb folgende Zusammensetzung:

| | |
|---|---|
| Monosekundärbutylamin | 84,5 % |
| Di-sekundärbutylamin | 9,9 % |
| Disekundärbutylether | 0,3 % |
| Sekundärbutanol | 4,7 % |
| Nebenprodukte | 0,4 % |

Dieses Beispiel zeigt, daß trotz ermäßigter Temperatur an einem kupferfreien, nickelreichen Katalysator in erhöhtem Maße Disekundärbutylether als ausbeuteminderndes Nebenprodukt auftritt.

Beispiel 7

Es wurde vorgegangen wie in Beispiel 4, jedoch mit dem Unterschied, daß die Zuflußmenge an Sekundärbutanol 2,0 1/1 x h betrug. Das Kondensat hatte nach 3-tägigem kontinuierlichem Betrieb folgende Zusammensetzung:

| | |
|---|---|
| Monosekundärbutylamin | 75,7 % |
| Di-sekundärbutylamin | 16,0 % |
| Sekundärbutanol | 7,2 % |
| Nebenprodukte | 1,1 % |

Dieses Beispiel zeigt die Möglichkeit ungewöhnlich hoher Raum-Zeit-Ausbeuten in der erfindungsgemäßen Verfahrens-Katalysator-Kombination.

Beispiel 9

Es wurde vorgegangen wie in Beispiel 4, jedoch mit dem Unterschied, daß 1 Liter Butanon (Methylethylketon) pro Liter Katalysator und pro Stunde dem auf 150 °C thermostatierten Reaktor zugeführt wurde. In einem einwöchigen kontinuierlichen Versuch hatte das Kondensat folgende mittlere Zusammensetzung:

| | |
|---|---|
| Monosekundärbutylamin | 84,7 % |
| Di-sekundärbutylamin | 10,0 % |
| Sekundärbutanol | 5,0 % |
| Nebenprodukte | 0,3 % |

Beispiel 10

Es wurde vorgegangen wie in Beispiel 1, jedoch mit dem Unterschied, daß 0,8 Liter Cyclohexanol pro Liter Katalysator und pro Stunde dem bei 220 °C thermostatierten Reaktor zugeführt wurden. In einem einwöchigen kontinuierlichen Versuch hatte das Kondensat folgende mittlere Zusammensetzung:

| | |
|---|---|
| Monocyclohexylamin | 95,7 % |
| Di-cyclohexylamin | 3,2 % |
| Cyclohexanol | 0,5 % |
| Nebenprodukte | 0,6 % |

Beispiel 11

Es wurde vorgegangen wie in Beispiel 1, jedoch mit dem Unterschied, daß n-Oktanol dem auf 210 °C thermostatierten Reaktor zugeführt wurde. Das n-Oktanol hatte einen Wertstoffgehalt von 98,5 %. In einem einwöchigen kontinuierlichen Versuch hatte das Kondensat folgende mittlere Zusammensetzung:

| | |
|---|---|
| Mono-n-oktylamin | 76,6 % |
| Di-n-oktylamin | 19,5 % |
| Tri-n-oktylamin | 1,0 % |
| n-Oktanol | 0,4 % |
| n-Heptan | 1,4 % |
| Nebenprodukte | 0,3 % |

Beispiel 12 (Vergleichsbeispiel)

Es wurde vorgegangen wie in Beispiel 11, jedoch mit dem Unterschied, daß ein kupferfreier Katalysator mit 65 Gew.-% Nickel verwendet wurde, und daß die Temperatur des Thermostatiermediums auf 180° eingestellt wurde. Das Kondensat hatte folgende mittlere Zusammensetzung:

| | |
|---|---|
| Mono-n-oktylamin | 77,6 % |
| Di-n-oktylamin | 15,5 % |
| Tri-n-oktylamin | 0,3 % |
| n-Oktanol | 1,2 % |
| n-Heptan | 4,3 % |
| Nebenprodukte | 1,1 % |

## Patentansprüche

**1.** Verfahren zur Herstellung von Aminen der allgemeinen Formel I

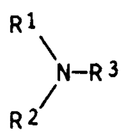

(I),

in der

$R^1$ Wasserstoff oder $R^2$,

$R^2$ $C_1$- bis $C_{12}$-Alkyl, $C_3$- bis $C_{12}$-Cycloalkyl, $C_2$- bis $C_{12}$-Alkoxyalkyl, gegebenenfalls durch $C_1$- bis $C_8$-Alkyl und/oder $C_1$- bis $C_8$-Alkoxy substituiertes Aryl oder $C_7$- bis $C_{12}$-Aralkyl,

$R^3$ Wasserstoff oder $C_1$- bis $C_{12}$-Alkyl

bedeutet, durch Umsetzung von Alkanolen der allgemeinen Formel II

$R^2$-OH (II),

in der $R^2$ die oben genannten Bedeutungen hat, mit Ammoniak oder einem primären Amin der allgemeinen Formel III

$R^3$-$NH_2$ (III),

in der $R^3$ die oben genannten Bedeutungen hat, und einem Katalysator bei Temperaturen von 100 bis 250°C und Drücken von 1 bis 100 bar, dadurch gekennzeichnet, daß man in der Gasphase arbeitet und Katalysatoren einsetzt, deren katalytisch aktive Masse 5 bis 100 Gew.-% eines Oxides von Kupfer und Nickel im Atomverhältnis von 1:1 bis 10:1 und Zirkon- und/oder Aluminiumoxid enthält.

**2.** Verfahren zur Herstellung von Aminen nach Anspruch 1, dadurch gekennzeichnet, daß man Katalysatoren einsetzt, deren katalytisch aktive Masse 20 bis 85 Gew.-% Zirkonoxid und/oder Aluminiumoxid, 1 bis 30 Gew.-% Kupferoxid, 1 bis 30 Gew.-% Nickeloxid und 1 bis 30 Gew.-% Kobaltoxid enthält.

**3.** Verfahren zur Herstellung von Aminen nach Anspruch 1, dadurch gekennzeichnet, daß man die

Reaktion in Anwesenheit von Wasserstoff und/oder Stickstoff durchführt.